# EUROPEAN PATENT APPLICATION

(11) **EP 1 956 002 A1**
(43) Date of publication of application: **13.08.2008**
(21) Application number: 07101855.0
(22) Date of filing: 07.02.2007
(51) Int. Cl.: C07D 209/14, A61K 31/4045, A61P 1/12

(54) **New tegaserod maleate polymorphs and process for their preparation**

(71) Applicant: Chemo Ibérica, S.A., 08028 Barcelona (ES)
(72) Inventor: Tacca, Alessandra, 28012, Cressa (Novara) (IT); Malpezzi, Luciana, 20131, Milano (IT)
(74) Representative: Barlocci, Anna

(57) **Abstract**

The present invention refers to two new tegaserod maleate polymorfs (α and β) and to processes for their preparation.

## Description

The present invention relates to polymorphic crystalline forms of the tegaserod maleate as well as to process for their preparation and their therapeutic use.

### BACKGROUND ART

Tegaserod maleate is a selective serotonin 5HT4-receptor partial agonist used in the treatment of irritable bowel syndrome. Its international nonpropietary name is 3-(5-methoxy-1 H-indole-3-yl-methylene)-N-pentylcarbazimidamide monomaleate, and corresponds to formula (1):

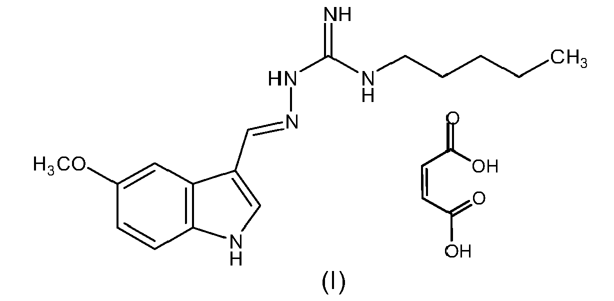

Tegaserod is disclosed in U.S. Patent No. 5.510.353 A.

There are known in the state of the art various tegaserod maleate polymorphs.

The Chinese patent CN 1.176.077 describes tegaserod maleate monohydrate crystals.

International publication WO 2004/085393 describes the crystalline Form I, II, III and IV of tegaserod maleate and processes for the preparation thereof. Particularly, Form IV of tegaserod is characterized by an x-ray powder diffraction pattern having peaks expressed as 2θ at about 6.9, 8.0, 10.3, 16.5, 19.6, 20.4, 20.9, 22.0, 23.2, 25.4, 28.0 and 28.7 degrees. Its preparation process comprises: a) mixing maleic acid and a solution of tegaserod free base in MeOH; and b) precipitating tegaserod maleate Form IV by mixing with methylene dichloride or isopropyl alcohol.

### SUMMARY OF THE INVENTION

The problem to be solved is the provision of new stable crystalline forms of tegaserod maleate.

The U. S. Food and Drug Administration recommends that an applicant for drug product marketing approval investigates whether the drug substance can exist in polymorphic forms. This is because the chemical and physical properties of the various forms can differ significantly and thereby have effects on drug product stability, dissolution characteristics, bioavailability, etc.

However, compliance with this recommendation is not a simple matter. Due to the influence of crystal form on formulation processing steps and the biological effects of a drug product, the preparation of crystalline forms of tegaserod maleate is desirable.

The solution is based on the provision of two new polymorphs of the tegaserod maleate (hereinafter referred as form α and form β).

The discovery of these two new crystalline forms of tegaserod maleate provides an opportunity to improve the performance characteristics of a pharmaceutical product. It enlarges the repertoire of materials that a formulation scientist has available for designing, for example, a pharmaceutical dosage form of a drug with a targeted release profile or other desirable characteristic.

Moreover, the solid state physical properties of tegaserod maleate can be influenced by controlling the conditions under which tegaserod maleate is obtained in solid form. Solid state physical properties include, for example, the flowability of the milled solid. Flowability affects the ease with which the material is handled during processing into a pharmaceutical product. When particles of the powdered compound do not flow past each other easily, a formulation specialist must take that fact into account in developing a tablet or capsule formulation, which may necessitate the use of glidants such as colloidal silicon dioxide, talc, starch or tribasic calcium phosphate.

Another important solid state property of a pharmaceutical compound is its rate of dissolution in aqueous fluid. The rate of dissolution of an active ingredient in a patient's stomach fluid can have therapeutic consequences since it imposes an upper limit on the rate at which an orally-administered active ingredient can reach the patient's bloodstream.

These practical physical characteristics are influenced by the conformation and orientation of molecules in the unit cell, which defines a particular polymorphic form of a substance. The polymorphic form may give rise to thermal behavior different from that of the amorphous material or another polymorphic form. Thermal behavior can be used to distinguish a polymorphic form from other but also a particular polymorphic form may also give rise to distinct spectroscopic properties that may be detectable by powderX-ray crystallography, solid state 13C NMR spectrometry and infrared spectrometry.

Thus, in a first aspect the present invention relates to tegaserod maleate Form α which is characterized by an X-Ray Powder Diffraction pattern having peaks at 2θ = 7.9, 10.2, 11.6, 16.0, 19.5, 23.2, 25.3, 27.9 ± 0.2.

In a second aspect the present invention relates to tegaserod maleate Form which is characterized by an X-Ray Powder Diffraction pattern having peaks at 2θ = 8.0, 8.7, 10.2, 11.9, 13.8, 15.5, 16.1, 17.1, 19.6, 23.1, 25.3, 27.0 ± 0.2.

In a third aspect the present invention relates to a process for preparing the tegaserod maleate form α according to the first aspect of the invention which comprises the steps of: a) suspending (+)-camphor-10-sulfonate addition salt of tegaserod in a polar medium in the presence of maleic acid; and b) recovering the resulting tegaserod maleate.

The present inventors have found that the use of a (+)-camphor-10-sulfonate addition salt of tegaserod (TEG-CSA) as the starting product is decisive for obtaining the form α of the present invention. In fact, when the suspension contains the (+)-camphor-10-sulfonate addition salt of tegaserod along with maleic acid in the presence of a polar medium, a exchange reaction occurs: camphorsulphonate anion is replaced by maleate anion, achieving the form α of the present invention. Without whishing to be bound by theory, it is believed by the present inventors that the already established crystalline order of the (+)-camphor-10-sulfonate addition salt of tegaserod is determining the crystalline structure of the final salt (in this case the maleate).

In a fourth aspect the present invention refers to a process for preparing the tegaserod maleate form β according to the second aspect of the invention which comprises the step of desolvating the tegaserod maleate form α, according to the first aspect of the invention, at a temperature ranging from 60-65 °C under reduced pressure.

Finally, the present invention also relates to pharmaceutical preparations comprising the tegaserod maleate form α or tegaserod maleate form β, or a mixture of both forms. The invention relates in particular to corresponding pharmaceutical preparations for the treatment of irritable bowel syndrome, gastro-esophageal reflux disease, functional dyspepsia, chronic constipation or diarrhea and subindications thereof. The invention relates to the use of the forms α or β of tegaserod maleate for the preparation of pharmaceutical preparations, in particular for the treatment of irritable bowel syndrome, gastro-esophageal reflux disease, functional dyspepsia, chronic constipation or diarrhea and subindications thereof.

Throughout the description and claims the word "comprise" and variations of the word, such as "comprising", is not intended to exclude other technical features, additives, components, or steps. The content of the application from which priority is claimed, as well as the contents of the abstract of the priority application and the present application, are incorporated herein as reference. Additional objects, advantages and features of the invention will become apparent to those skilled in the art upon examination of the description or may be learned by practice of the invention.

### BRIEF DESCRIPTION OF THE DRAWINGS

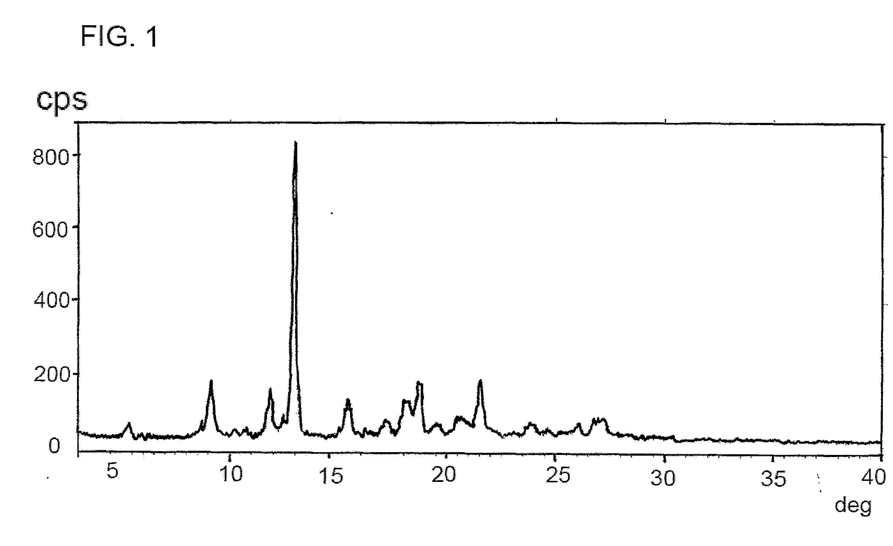

FIG. 1 is the X-Ray Powder diffractogram of the TEG-CSA salt having characteristic diffraction peaks at 2θ = 9.1, 11.8, 13.0, 15.4, 18.7, 22.5 ± 0.2.
FIG. 2 is the X-Ray Powder diffractogram of tegaserod maleate form α having characteristic diffraction peaks at 2θ = 7.9, 10.2, 11.6, 16.0, 19.5, 23.2, 25.3, 27.9 ± 0.2
FIG. 3 is the X-Ray powder diffractogram of tegaserod maleate form β having the characteristic diffraction peaks at 2θ = 8.0, 8.7, 10.2, 11.9, 13.8, 15.5, 16.1, 17.1, 19.6, 23.1, 25.3, 27.0 ± 0.2.

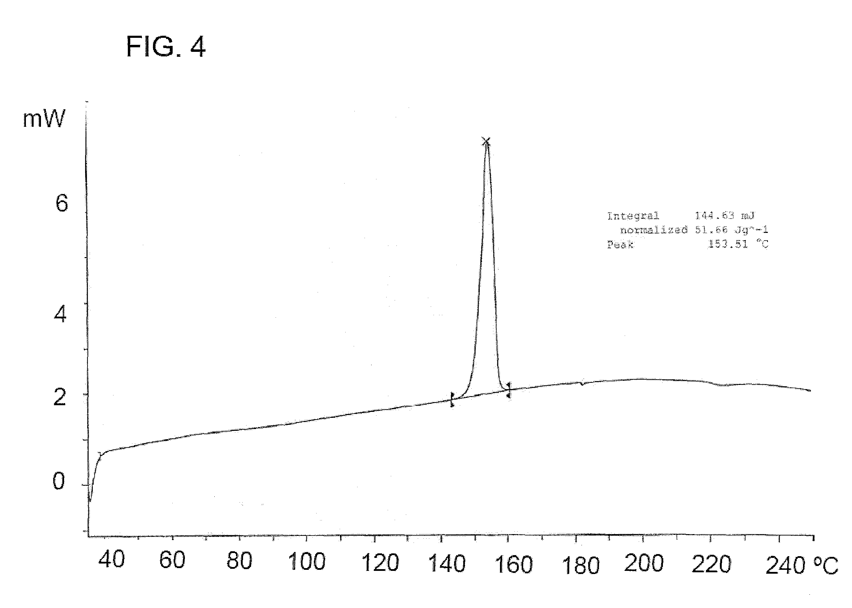

FIG. 4 is the DSC thermal of TEG-CSA salt which shows a broad endothermic around 154 °C.

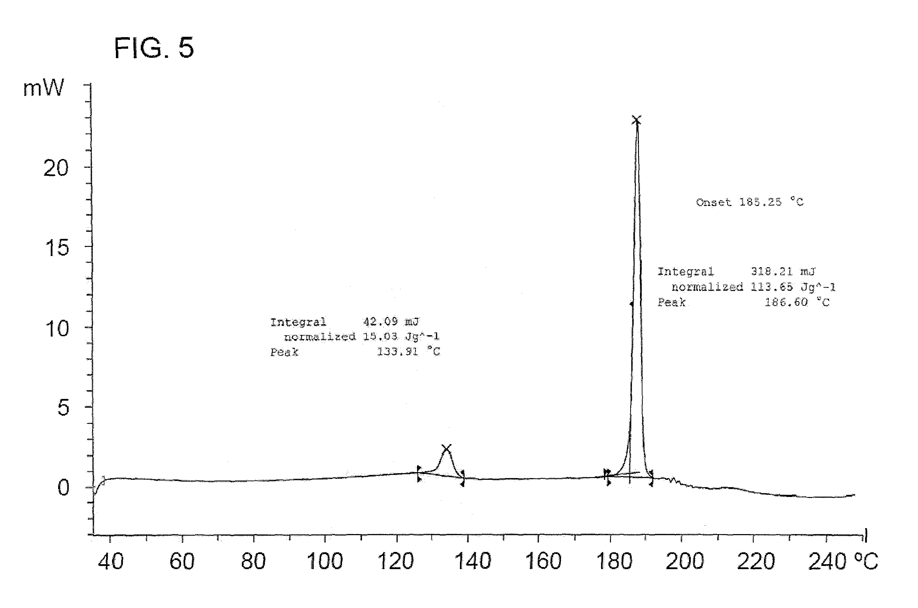

FIG. 5 is the DSC thermal of tegaserod maleate form α, which shows a broad endothermic peak around 134°C and an endothermic peak at 187 °C.

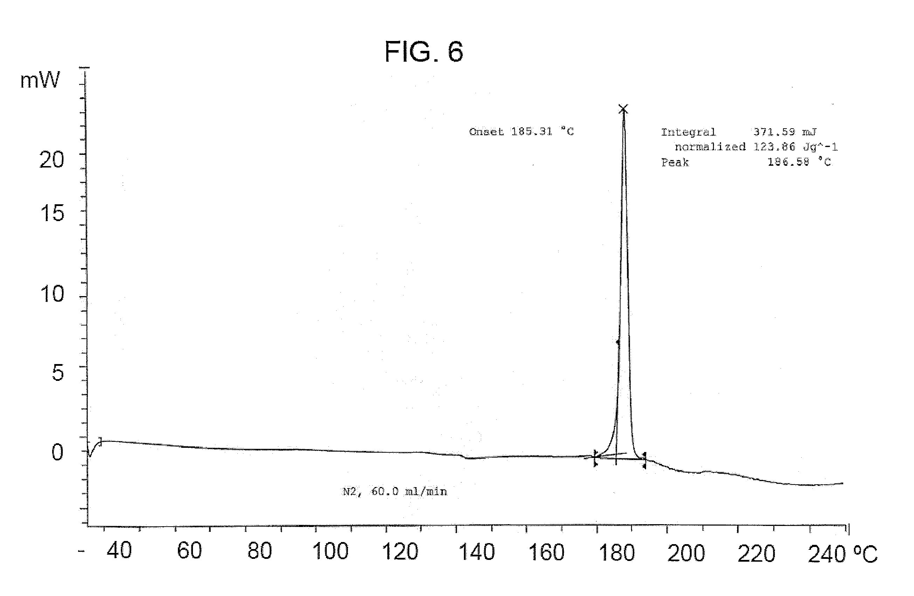

FIG. 6 is the DSC thermal of tegaserod maleate form β, which shows only an endothermic peak around 187 °C.

TEG-CSA salt and Tegaserod maleate forms α and β have been characterized by DSC and XRPD.

XRPD diffraction spectrum was performed on a APD 2000 Ital Structures diffraction meter at room temperature, using a CuKα tube (40 kV, 30 mA) as the x-ray source. Data collection was done in 2θ continuous scan mode, at a scan speed of 0.02°/s in the range of 3° to 40° in 2θ. Approximately 100 mg samples were grinded and placed on an aluminium sampler.
DSC thermal analysis was performed on a Mettler Toledo Star 822^{e} differential scanning calorimeter. Approximately 2-5 mg samples were placed in aluminium pans and heated from 35 to 250 °C in a dry nitrogen atmosphere at a heating rate of 10°C/minute.

### DETAILED DESCRIPTION OF PARTICULAR EMBODIMENTS

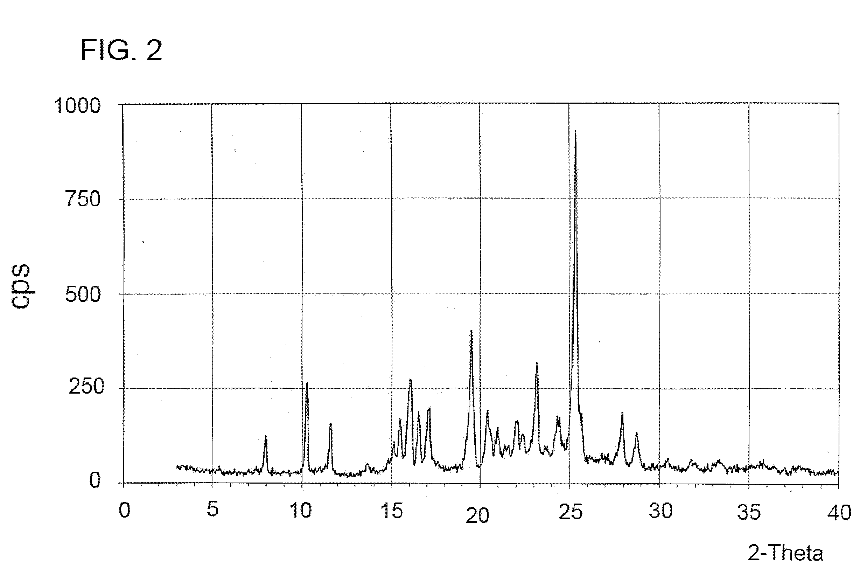

Preferably the tegaserod maleate Form α substantially corresponds to the X-Ray diffractogram of FIG. 2.

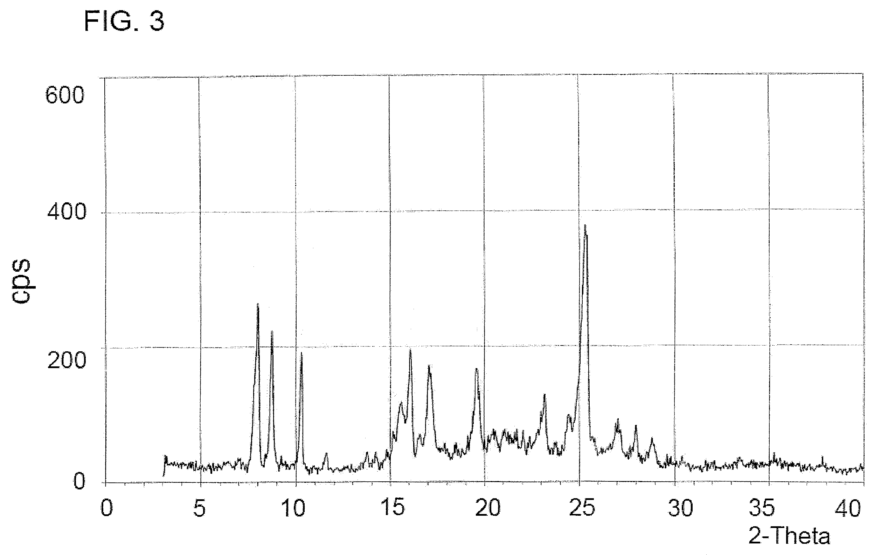

Preferably the tegaserod maleate Form β substantially corresponds to the X-Ray diffractogram of FIG. 3.

In a preferred embodiment of the third aspect of the invention the polar medium of step (a) is selected from the group consisting of: methylene chloride, water and mixtures thereof.

In still another preferred embodiment of the third aspect of the invention the amount of maleic acid added in step (a) is ranging from 1 to 3 moles per mol of tegaserod free base, preferably said amount is ranging from 1.5 to 2.5 moles of maleic acid per mol of tegaserod free base.

In still yet another preferred embodiment, the (+)-camphor-10-sulfonate addition salt of tegaserod is obtained following the steps of: i) reacting tegaserod free base with (+)-camphor-10-sulfonic acid in a polar medium to obtain the corresponding (+)-camphor-10-sulfonate addition salt; and ii) recovering the addition salt.

Preferably, the polar medium of step (i) is selected from the group consisting of: N,N-dimethylacetamide, N,N-dimethylformamide and mixtures thereof.

The forms α or β (or mixtures of α and β forms of tegaserod maleate) can be used, for example, in the form of pharmaceutical preparations which comprise a therapeutically effective amount of the active ingredient, if desired together with inorganic or organic, solid or liquid, pharmaceutically usable carriers, which are suitable for enteral, for example oral, or parenteral administration. Furthermore, the forms α and β of tegaserod maleate can be used in the form of preparations which can be administered parenterally or in the form of infusion solutions. The pharmaceutical preparations may be esterilized and/or may comprise excipients, for example preservatives, stabilizers, wetting agents and/or emulsifiers, solubilizers, salts for regulating the osmotic pressure and/or buffers.

The following examples and drawings are provided by way of illustration, and are not intended to be limiting of the present invention.

### EXAMPLES

### Example 1 - Preparation of tegaserod maleate form α

To a solution of tegaserod free base (87 g, 288 mmol) in 360 ml dimethylformamide at 45 °C, 81 g of (S)-(+)-camphor-10-sulfonic acid (349 mmol) were added. The resulting mixture was cooled to 20 °C, and then 600 ml of water were added to precipitate the addition salt. The product was isolated by filtration and washed with water.

The wet product (300 g) was suspended under stirring in 450 ml CH₂Cl₂, and 50 g of maleic acid (430 mmol) in 100 ml of water were added. The resulting mixture was stirred at 30-35 °C for a hour. Then 50 g of maleic acid and 400 ml of water were added. After cooling to ambient temperature, tegaserod maleate form α, hemisolvate with methylene chloride, was collected by filtration, washed with methylene chloride, water and acetone, and dried under reduced pressure at 40 °C. 120 g of product were obtained (90% yield; ¹H-NMR, Jeol Eclipse 300, DMSO-d₆: δ=11.60 ppm, s, 1 H; 11.00 ppm, s, 1 H; 8.35 ppm, s, 1 H; 7.85 ppm, d, J=2.76 Hz, 1 H; 7.79 ppm, t, J=5.49 Hz, 1 H; 7.68 ppm, m, 1 H; 7.36 ppm, d, J=8.88 Hz, 1 H; 6.86 ppm, dd, J= 8.88 Hz, J=2.76 Hz, 1 H; 6.08 ppm, s, 2H; 5.76 ppm, s, 1 H (methylene chloride); 3.83 ppm, s, 3H; 3.29 ppm, m, 2H; 1.57 ppm, m, 2H; 1.32 ppm, m, 4H; 0.88 ppm, t, 3H, J=6.75 Hz).

### Example 2 - Preparation of tegaserod maleate form β

10 g of tegaserod maleate form a were accurately grinded and placed in an oven at 65 °C under reduced pressure (2 mm Hg) for three days. 9 g of tegaserod maleate form β were obtained.

## Claims

1. Tegaserod maleate Form α which is **characterized by** an X-Ray Powder Diffraction pattern having peaks at 2θ = 7.9, 10.2, 11.6, 16.0, 19.5, 23.2, 25.3, 27.9 ± 0.2.

2. Tegaserod maleate Form α as claimed in claim 1 which substantially corresponds to the X-Ray diffractogram of FIG. 2.

3. Tegaserod maleate Form β which is **characterized by** an X-Ray Powder Diffraction pattern having peaks at 2θ = 8.0, 8.7, 10.2, 11.9, 13.8, 15.5, 16.1, 17.1, 19.6, 23.1, 25.3, 27.0 ± 0.2.

4. Tegaserod maleate Form β as claimed in claim 2 which substantially corresponds to the X-Ray diffractogram of FIG. 3.

5. A process for preparing the tegaserod maleate form α as claimed in any of claims 1-2 which comprises the steps of:
a) suspending (+)-camphor-10-sulfonate addition salt of tegaserod in a polar medium in the presence of maleic acid; and
b) recovering the resulting tegaserod maleate.

6. The process as claimed in claim 5 wherein the polar medium of step (a) is selected from the group consisting of: methylene chloride, water and mixtures thereof.

7. The process as claimed in claim 5 wherein the amount of maleic acid added in step (a) is ranging from 1 to 3 moles per mol of tegaserod free base.

8. The process as claimed in claim 7 wherein the amount is ranging from 1.5 to 2.5 moles of maleic acid per mol of tegaserod free base.

9. The process as claimed in claim 5 wherein the addition salt of tegaserod is obtained following the steps of:
i) reacting tegaserod free base with (+)-camphor-10-sulfonic acid in a polar medium to obtain the corresponding (+)-camphor-10-sulfonate addition salt; and
ii) recovering the addition salt.

10. The process as claimed in claim 9 wherein the polar medium of step (i) is selected from the group consisting of: N,N-dimethylacetamide, N,N-dimethylformamide and mixtures thereof.

11. A process for preparing the tegaserod maleate form β as claimed in any of claims 3-4 which comprises the step of desolvating the tegaserod maleate form α as claimed in claims 1-2 at a temperature ranging from 60-65 °C under reduced pressure.

12. A pharmaceutical composition comprising a pharmaceutically acceptable carrier or diluent and a therapeutically effective amount of the tegaserod maleate form α as claimed in any of the claims 1-2.

13. A pharmaceutical composition comprising a pharmaceutically acceptable carrier or diluent and a therapeutically effective amount of the tegaserod maleate form β as claimed in any of the claims 3-4.

14. Use of tegaserod maleate form α as claimed in any of the claims 1-2 for the manufacture of a medicament for the treatment of irritable bowel syndrome, gastro-esophageal reflux disease, functional dyspepsia, chronic constipation or diarrhea.

15. Use of tegaserod maleate form β as claimed in any of the claims 3-4 for the manufacture of a medicament for the treatment of irritable bowel syndrome, gastro-esophageal reflux disease, functional dyspepsia, chronic constipation or diarrhea.
